# EUROPEAN PATENT APPLICATION

(11) **EP 4 064 121 A1**
(43) Date of publication of application: **28.09.2022**
(21) Application number: 22161843.2
(22) Date of filing: 14.03.2022
(51) Int. Cl.: G06K 9/00

(54) **POSTURE RECOGNITION SYSTEM, POSTURE RECOGNITION METHOD, AND PROGRAM**

(30) Priority: 24.03.2021 JP 2021050639
(71) Applicant: Hitachi, Ltd., Tokyo 100-8280 (JP)
(72) Inventor: NIIKURA, Takehiro, Tokyo, 100-8280 (JP); AKIYAMA, Takayuki, Tokyo, 100-8280 (JP); TAETZ, Bertram, 67663 Kaiserslautern (DE); BLESER, Gabriele, 67663 Kaiserslautern (DE); LORENZ, Michael, 67663 Kaiserslautern (DE); STRICKER, Didier, 67663 Kaiserslautern (DE)
(74) Representative: MERH-IP Matias Erny Reichl Hoffmann Patentanwälte PartG mbB

(57) **Abstract**

Provided is a posture recognition system that can properly respond to an error that is changed according to a user's motion.

A posture estimation section calculates posture data indicating a posture of a user wearing clothing, on the basis of motion data measured by a posture sensor attached to the clothing. An error estimation section calculates error data which is an estimate of an error that is occurring in the posture data, on the basis of the motion data and the posture data.

## Description

### BACKGROUND OF THE INVENTION

### 1. Field of the Invention

The present disclosure relates to a posture recognition system, a posture recognition method, and a program.

### 2. Description of the Related Art

A posture recognition system that recognizes a user's posture has drawn attention in recent years. A posture recognition system is used, for example, to find burdens on workers engaged in industrial or agricultural activities, ensure safety of the workers, or improve work efficiency, and can send an instruction according to a worker's posture to the worker.

A motion capture system that photographs a user by using photographing equipment such as camera and a close-contact type wearable sensor system that keeps a sensor such as an acceleration sensor capable of detecting a worker's motion in close contact with a user's body are known as posture recognition systems. With the motion capture system, however, the user must remain within a photographing range of the photographing equipment, which has put a limitation on a user's action. With the close-contact type wearable sensor system, there are also problems one example of which is that the sensor itself has been a limitation on a worker's action.

In contrast, JP-2009-18158-A discloses a technique that attaches a plurality of sensors such as temperature sensors and a plurality of pieces of wireless equipment to clothing and recognizes the user's posture from signals obtained by the respective sensors. This technique makes it possible to reduce limitations on the user's action.

In order to solve a problem of a posture recognition error that occurs in a case where a sensor is changed in its position due to slipping of clothing in a clothing type sensor system in which the sensor is attached to clothing, a technique is disclosed in "Shinya Namikawa, Yu Enokibori, Kenji Mase, "A study of preventive measures against misalignment of clothing type sensor by arranging a plurality of sensors of the same type in proximity," Multimedia, Distributed, Cooperative, and Mobile (DICOMO2016) Symposium, July 2016, p.1183-1189" in which a plurality of sensors are arranged in proximity and an optimal sensor is adopted from among these sensors.

In the technique described in JP-2009-18158-A, no consideration has been given to an error that occurs in the case where a sensor is attached to clothing. Although consideration has been given to an error that occurs when a sensor is changed in its position due to slipping of clothing in the technique described in "Shinya Namikawa, Yu Enokibori, Kenji Mase, "A study of preventive measures against misalignment of clothing type sensor by arranging a plurality of sensors of the same type in proximity," Multimedia, Distributed, Cooperative, and Mobile (DICOMO2016) Symposium, July 2016, p.1183-1189," what is done here is merely to select, from among the plurality of sensors arranged in proximity, the sensor that provides the smallest error that occurs in the case where a specific motion is made. Accordingly, it has been difficult to properly respond to an error whose cause and magnitude are changed according to the user's motion.

It is an object of the present disclosure to provide a posture recognition system, a posture recognition method, and a program that can properly respond to an error that is changed according to a user's motion.

### SUMMARY OF THE INVENTION

A posture recognition system according to an aspect of the present disclosure includes a posture estimation section and an error estimation section. The posture estimation section calculates, on the basis of sensor data measured by a posture sensor attached to clothing, posture data indicating a posture of a user wearing the clothing. The error estimation section calculates error data, which is an estimate of an error that is occurring in the posture data, on the basis of the sensor data and the posture data.

According to the present invention, it is possible to properly respond to an error that is changed according to a user's motion.

### BRIEF DESCRIPTION OF THE DRAWINGS

Fig. 1 is a block diagram illustrating a posture recognition system according to embodiment 1 of the present disclosure;
Fig. 2 is a schematic diagram illustrating an example in which a user wears clothing to which sensor sections are attached;
Fig. 3 is a conceptual diagram for describing an example of a function of a posture estimation section;
Figs. 4A to 4D are diagrams for describing examples of errors that occur in an estimated posture;
Fig. 5 is a diagram illustrating examples of classes into which an error is classified;
Fig. 6 is a flowchart for describing operation of the posture recognition system;
Figs. 7A and 7B are diagrams for describing examples of errors according to a posture sensor position; and
Figs. 8A and 8B are diagrams for describing other examples of errors according to the posture sensor position.

### DESCRIPTION OF THE PREFERRED EMBODIMENTS

A description will be given below of embodiments of the present disclosure with reference to drawings. It should be noted, however, that the following embodiments are illustrative for description of the present disclosure and are not intended to limit the scope of the present disclosure to these embodiments. A person skilled in the art can carry out the present disclosure in various other manners without departing from the scope of the present disclosure.

In the configuration of the invention described below, the same portions or portions having the same function may be denoted by the same reference signs in common in different drawings for omission of redundant description. In the case where there are a plurality of elements having the same or similar functions, such elements may be described by adding different suffixes to the same reference sign. It should be noted, however, that in the case where there is no need to distinguish between the plurality of elements, such elements may be described by omitting suffixes.

Actual positions, sizes, shapes, and regions of the components illustrated in the drawings may not be given to facilitate the understanding of the present disclosure. Accordingly, the present disclosure is not limited to the positions, sizes, shapes, and regions disclosed in the drawings. Even if a component is illustrated in singular form in the present specification, there may be the plurality of components unless otherwise indicated in context.

### [Embodiment 1]

### <1. Overall Configuration of the System>

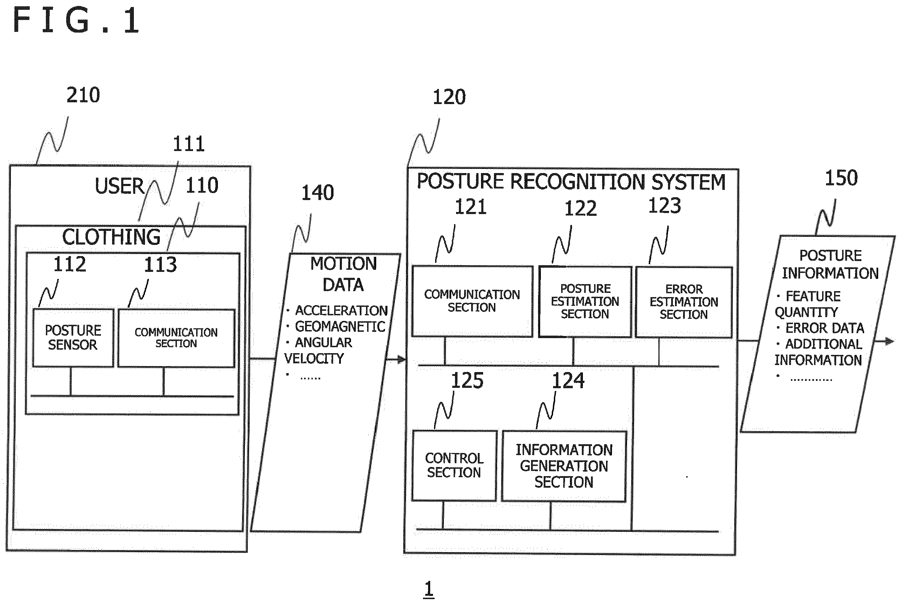

Fig. 1 is a block diagram illustrating a posture recognition system according to embodiment 1 of the present disclosure. A posture recognition system 1 illustrated in Fig. 1 estimates a posture of a user 210 and includes a sensor section 110 and an information processing apparatus 120. The sensor section 110 and the information processing apparatus 120 are connected to each other in a wired or wireless manner for communication. It should be noted that the sensor section 110 and the information processing apparatus 120 may be connected to each other via a communication network.

The sensor section 110 is a wearable unit attached to clothing 111 of the user 210. The sensor section 110 may be, for example, pasted to the clothing 111 by using adhesive tape or embedded in advance in the clothing 111. The clothing 111 is, for example, common clothing that is not in close contact with a body of the user 210 such as work clothing.

The sensor section 110 includes a posture sensor 112 and a communication section 113. The posture sensor 112 is a motion measurement sensor for measuring the motions of the user 210. The motions refer to motions in general including not only motions associated with industrial and agricultural activities but also motions for specific purposes such as dancing, gymnastics, and performance with musical instruments.

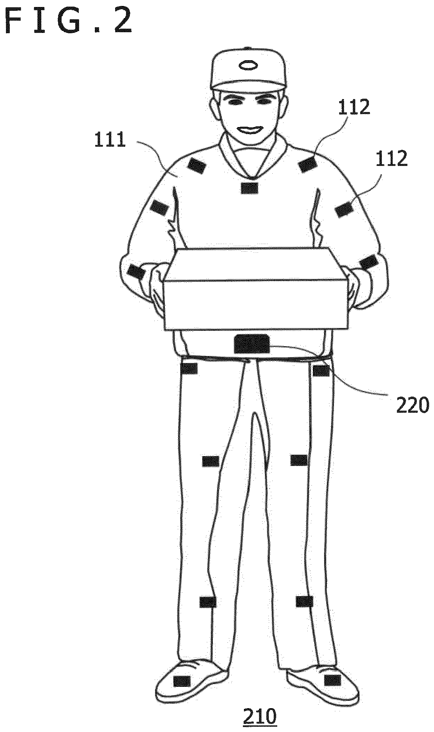

Fig. 2 is a schematic diagram illustrating an example in which the user 210 is wearing the clothing 111 to which the sensor section 110 is attached.

In Fig. 2, the clothing 111 has the plurality of posture sensors 112 as the sensor sections 110 and a sensor hub 220 that collects motion data 140 (refer to Fig. 1) which is sensor data measured by the plurality of posture sensors 112. Each of the posture sensors 112 is connected to the sensor hub 220 directly or by way of other posture sensor 112 and sends the motion data 140 to the sensor hub 220. It should be noted that the posture sensors 112 and the sensor hub 220 may be connected to each other in a wireless or wired manner.

Although the posture sensors 112 are attached to the clothing 111 in such a manner as to be arranged on respective body parts of the user 210 in the example illustrated in Fig. 2, the arrangement of the posture sensors 112 is not limited to this example. For example, the posture sensors 112 may be attached to the clothing 111 in such a manner as to be arranged on only some body parts of the user such as upper or lower body.

Only one type or a plurality of types of the posture sensors 112 may be used, and the type of the posture sensors 112 may be selected as appropriate according to a target's posture to be estimated. For example, in the case where the posture of the user 210 is directly estimated, it is preferred that the type of sensors that can measure the positions or motions of the respective body parts of the user 210 such as acceleration sensor or position sensor be used. The type of the posture sensors 112 is not limited to this example, and gyro sensor, geomagnetic sensor, myoelectric sensor, acceleration sensor, video sensor, or audio sensor, for example, may also be used.

The sensor hub 220 sends the motion data 140 from the respective posture sensors 112 to the information processing apparatus 120 via the communication section 113. Although not illustrated in Fig. 2, the communication section 113 is incorporated, for example, in the sensor hub 220. It may be possible to feed power to the posture sensors 112 and the sensor hub 220 from a power feeding apparatus (not illustrated) such as battery.

Referring back to the description of Fig. 1, the information processing apparatus 120 functions as a posture estimation apparatus that estimates the posture of the user 210 on the basis of the motion data 140 from the sensor section 110. The information processing apparatus 120 can include a common server. For example, the information processing apparatus 120 can include a server that includes an input apparatus, an output apparatus, a processing apparatus, and a storage apparatus as hardware. In the present embodiment, a program stored in the storage apparatus is read and executed by the processing apparatus, as a result of which a variety of functions are realized due to coordination between the processing apparatus and other hardware as necessary.

It should be noted that the information processing apparatus 120 may include a single server or other computer system in which optional portions of the input apparatus, the output apparatus, the processing apparatus, and the storage apparatus are connected to each other by a network. Functions equivalent to those realized by the above program may be realized by hardware such as FPGA (Field Programmable Gate Array) or ASIC (Application Specific Integrated Circuit). A neural network which will be described later may be realized by FPGA.

The information processing apparatus 120 includes a communication section 121, a posture estimation section 122, an error estimation section 123, an information generation section 124, and a control section 125 as functional components.

The communication section 121 communicates with external apparatuses such as the sensor section 110. For example, the communication section 121 receives the motion data 140 from the communication section 113 of the sensor section 110. The communication section 121 sends posture information 150 indicating the posture of the user 210 estimated on the basis of the motion data 140. Although destinations of the posture information 150 are not specifically limited, the posture information 150 is sent, for example, to the storage apparatus that stores the posture information 150, a display apparatus that displays the posture information 150, or an analysis apparatus that analyzes the posture information 150.

The posture estimation section 122 estimates the posture of the user 210 on the basis of the motion data 140 received by the communication section 121 and calculates posture data indicating the estimated posture which is an estimate of the posture. The error estimation section 123 estimates an error that is occurring in the posture data calculated by the posture estimation section 122 and calculates error data indicating the estimated error. The posture estimation section 122 and the error estimation section 123 can be configured, for example, by using a neural network.

The information generation section 124 generates, on the basis of the posture data calculated by the posture estimation section 122 and the error data calculated by the error estimation section 123, additional information which is an evaluation of the error data. The control section 125 controls the information processing apparatus 120 as a whole.

### <2. Posture Estimation Section>

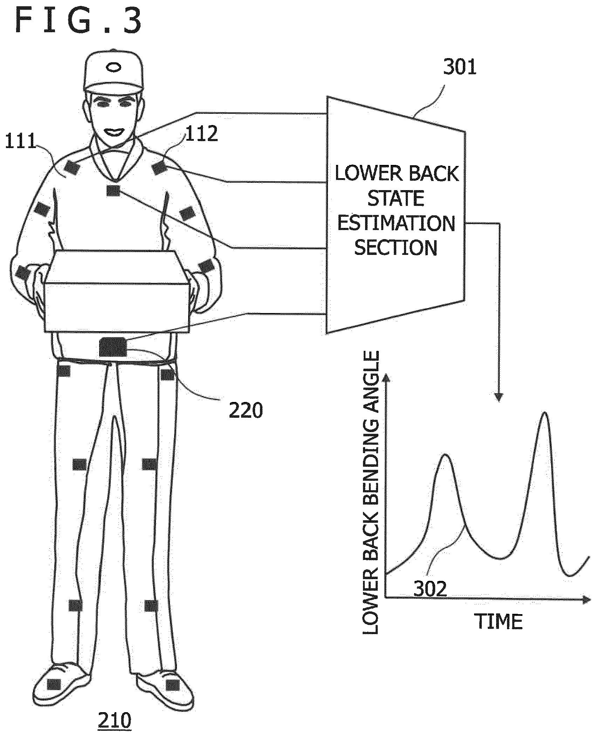

Fig. 3 is a conceptual diagram for describing an example of a function of the posture estimation section 122. The posture estimation section 122 calculates posture data indicating the estimated posture of the user 210 as described above. The estimated posture specifically refers to an estimate of the posture or postures of one or a plurality of given body parts of the user 210. The body part refers to a body part that can be considered as a single rigid body (e.g., upper arm, forearm, or shin).

In the present embodiment, the posture estimation section 122 calculates a feature quantity representing a feature of the estimated posture as posture data. The feature quantities correspond to states of moving parts (joints) connected to body parts and are, for example, elbow bending angle, lower back bending angle, head orientation, and opening leg angle.

The type and arrangement of the posture sensors 112 are selected according to the target's posture to be estimated. A description will be given below of an example in which a lower back bending angle is calculated as a feature quantity which is posture data by using an acceleration sensor as the posture sensor 112 unless otherwise specified. In this case, the posture estimation section 122 includes a lower back state estimation section 301 that calculates a lower back bending angle as illustrated in Fig. 3.

The lower back state estimation section 301 calculates, for example, a rotation angle of the lower back in a fore-and-aft direction as a bending angle of the lower back which is a feature quantity on the basis of the motion data 140 from the four posture sensors 112 arranged on shoulders, a chest, and the lower back of the user 210 illustrated in Fig. 3. Since values of the motion data 140 are changed over time, the lower back state estimation section 301 acquires the lower back bending angle as chronological data 302. The acquired chronological data 302 may be stored, for example, in the storage apparatus within the information processing apparatus 120 or in an external storage apparatus.

The lower back state estimation section 301 includes, for example, a learning model built by known supervised learning by using a deep neural network (hereinafter referred to as a DNN) that has the motion data 140 as an explanatory variable and a feature quantity as an objective variable. It should be noted that the lower back state estimation section 301 may calculate a feature quantity from the motion data 140 of the posture sensors 112 without using a DNN. For example, the lower back state estimation section 301 can estimate the lower back bending angle by acquiring, in advance, the motion data 140 (acceleration) in a given posture (e.g., upright posture) of the user as an initial state or by acquiring the motion data 140 of other sensor such as position sensor different from the acceleration sensor without using a DNN.

It should be noted that in the case where a feature quantity other than the lower back bending angle is calculated, it is only necessary to add, in keeping with the feature quantity, a functional section such as elbow rotation angle estimation section or head orientation estimation section which calculates the feature quantity to the posture estimation section 122.

### <3. Error Estimation Section>

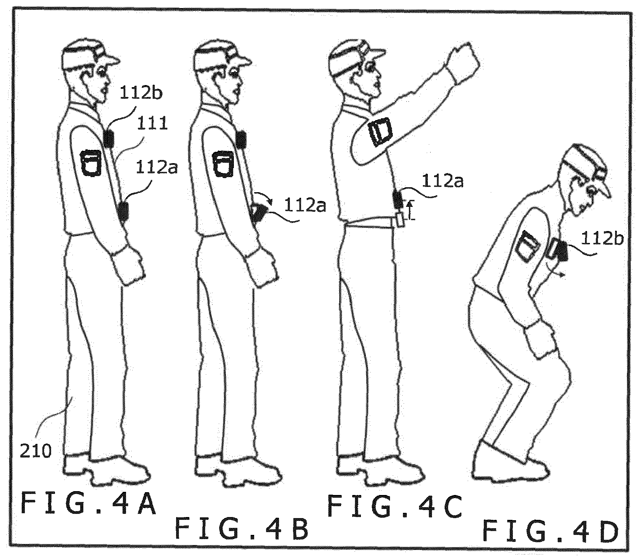

Figs. 4A to 4D are diagrams for describing examples of errors that occur in posture data (feature quantity) calculated by the posture estimation section 122 due to deformation of the clothing 111.

Normally, the posture sensors 112 are attached to the clothing 111 such that the positions and orientations thereof with respect to the body of the user 210 are as expected in advance as illustrated in Fig. 4A. However, in the case where the user 210 makes some kind of motion, or in the case where the clothing 111 does not partially fit a body shape of the user 210, the positions and orientations of the posture sensors 112 may deviate from the expected positions and orientations.

For example, in the case where there is a slight slack in the clothing 111 on an abdomen as illustrated in Fig. 4B, there is a possibility that a minor error may occur due to slight deviation in the orientation of the posture sensor 112a which is the posture sensor 112 arranged on the abdomen. In the case where the user 210 extends his or her arm upward as illustrated in Fig. 4C, a hem of the clothing 111 is raised in keeping with the movement of the arm, which may cause the position of the posture sensor 112a arranged on the abdomen to deviate and result in a medium error. In the case where the user tilts forward as illustrated in Fig. 4D, the posture sensor 112b which is the posture sensor 112 arranged on the chest tilts in keeping with the forward tilting of the user 210 if the posture sensor 112b is in close contact with the body of the user 210. However, in the case where the posture sensor 112b is attached to the clothing 111, the posture sensor 112b may remain approximately vertical relative to the ground due to deformation of the chest of the clothing 111 (e.g., slack). In this case, there is a possibility that a large error may occur because no information can be acquired that indicates that the user 210 is tilting forward from the motion data 140 of the posture sensor 112b.

In the case where the posture sensors 112 are attached to the clothing 111 as described above, an error may occur in posture data calculated by the posture estimation section 122 due to deformation of the clothing 111. The error estimation section 123 calculates error data which is an estimate of an error that is occurring in the posture data on the basis of the motion data 140 and the posture data. It should be noted that since error data is changed over time, the error estimation section 123 acquires an error as chronological data. The acquired chronological data may be stored, for example, in the storage apparatus within the information processing apparatus 120 or in an external storage apparatus.

The error estimation section 123 includes, for example, a learning model built by known supervised learning by using a DNN that has the motion data 140 and posture data as explanatory variables and an error as an objective variable. A learning model can be built, for example, by causing the user 210 wearing the clothing 111 to which the posture sensors 112 are attached to perform various tasks, acquiring an error between a user's actual posture and an estimated posture (posture data), and using known supervised learning in which the motion data 140, posture data, and error are used as training data.

It should be noted that the motion data 140 used as training data can be selected as appropriate according to the target's posture to be estimated and the body part. For example, in the case where the lower back bending angle is estimated, the motion data 140 used as training data may be motion data from the posture sensors 112 arranged on the abdomen and chest or data obtained by adding motion data from the posture sensors 112 arranged on the shoulders and upper arms to the motion data from the posture sensors arranged on the abdomen and chest. In the case where the lower back bending angle is estimated, posture data used as training data may be the lower back bending angle or data obtained by adding feature quantities associated with the upper arms to the lower back bending angle.

As means of acquiring the user's actual posture, the actual posture may be visually determined from a video image acquired by using imaging equipment such as camera. Alternatively, a known motion capture system or a close-contact type wearable sensor system may be used.

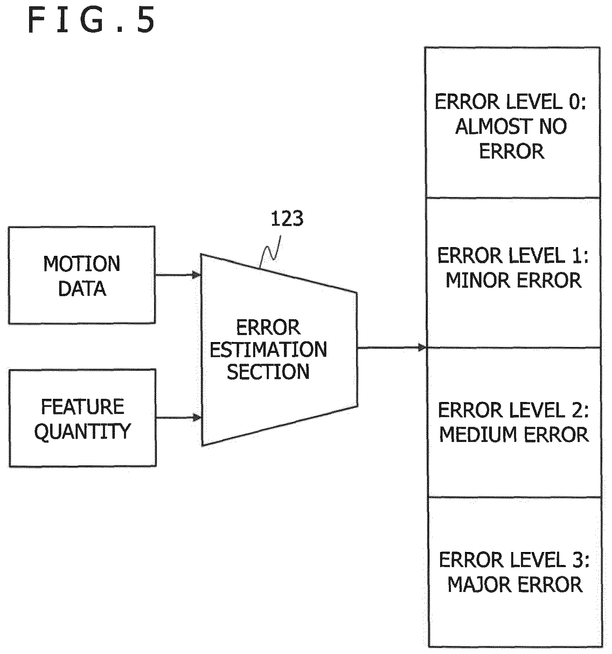

The error estimation section 123 may estimate an error by classifying the error into a plurality of classes. Fig. 5 is a diagram illustrating examples of classes into which an error is classified. In the example illustrated in Fig. 5, an error is classified into four classes from error level 0 to error level 3. Error level 0 is a class with almost no error. Error level 1 is a class with a minor error. Error level 2 is a class with a medium error. Error level 3 is a class with a major error.

### <4. Information Generation Section>

The information generation section 124 generates additional information which is an evaluation of error data on the basis of posture data calculated by the posture estimation section 122 and error data calculated by the error estimation section 123 and generates the posture information 150 that includes the posture data, the error data, and the additional information.

Additional information is, for example, evaluation information indicating availability or reliability of the estimated posture or notification information according to an error. The evaluation information refers specifically to information indicating the extent to which an estimated posture is reliable and may be calculated by using an error itself or by using a statistical value such as time average of an error. Notification information is, for example, information for notifying the user 210 or an administrator of the posture recognition system 1 that an error is large in the case where the error or the statistical value thereof is equal to or larger than a threshold. Notification information may be, for example, output as an alert tone from the information processing apparatus held by the user 210 or the administrator of the posture recognition system 1.

### <5. Operation>

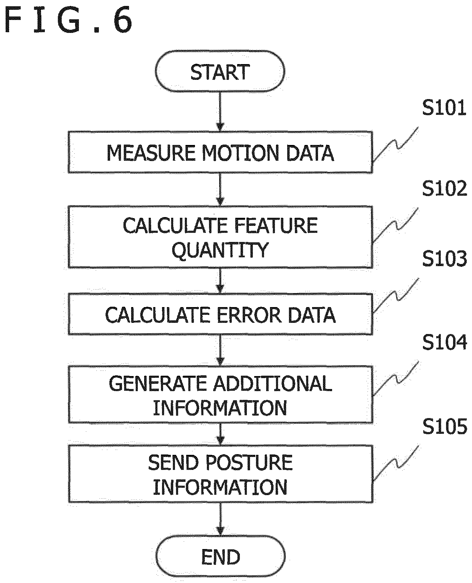

Fig. 6 is a flowchart for describing operation of the posture recognition system 1.

First, each of the posture sensors 112 of the sensor section 110 regularly or continuously measures the motion data 140 and outputs the motion data 140 to the sensor hub 220. The sensor hub 220 sends the motion data 140 sent from each of the posture sensors 112 to the information processing apparatus 120 via the communication section 113 (step S101).

When the communication section 121 of the information processing apparatus 120 receives the motion data 140, the posture estimation section 122 estimates the posture of the user 210 on the basis of the motion data 140 and calculates a feature quantity of the estimated posture as posture data indicating the estimated posture (step S102).

The error estimation section 123 estimates an error that is occurring in the feature quantity calculated by the posture estimation section 122 on the basis of the motion data 140 and the feature quantity and calculates error data that indicates the estimated error (step S103).

The information generation section 124 generates additional information which is an evaluation of the error data on the basis of the feature quantity and the error data (step S104).

The information generation section 124 generates the posture information 150 that includes the feature quantity, the error data, and the additional information. The communication section 121 sends the posture information (step S105) and terminates the process.

### <6. Advantageous Effect of the Embodiments>

According to the embodiment described above, the posture estimation section 122 calculates posture data indicating the posture of the user 210 wearing the clothing 111 on the basis of the motion data 140 measured by the posture sensors 112 attached to the clothing 111. The error estimation section 123 calculates error data which is an estimate of an error that is occurring in the posture data on the basis of the motion data 140 and the posture data. Accordingly, it becomes possible to recognize the cause and magnitude of the error that are changed according to the motion of the user 112 due to attachment of the posture sensors 112 to the clothing 111, which makes it possible to properly respond to the error. For example, it becomes possible for an application program that performs a variety of processes on the basis of posture data to perform the processes in consideration of the error, which makes it possible to perform the processes on the basis of the posture data with more accuracy.

In the present embodiment, posture data and error data are chronological data. Accordingly, it becomes possible to find the change in error over time, which makes it possible to more properly respond to the error.

In the present embodiment, the information generation section 124 generates additional information which is an evaluation of error data. Accordingly, it becomes possible to issue notification about reliability of posture data, which makes it possible to properly respond to an error with ease.

### [Embodiment 2]

In the present embodiment, a description will be given of an example of the posture recognition system 1 that can reduce an error by generating posture data and error data corresponding to each of the plurality of posture sensors 112 attached to different positions. The posture sensors 112 are specifically attached to the clothing 111 in such a manner as to be arranged on a plurality of different body parts of the user 210.

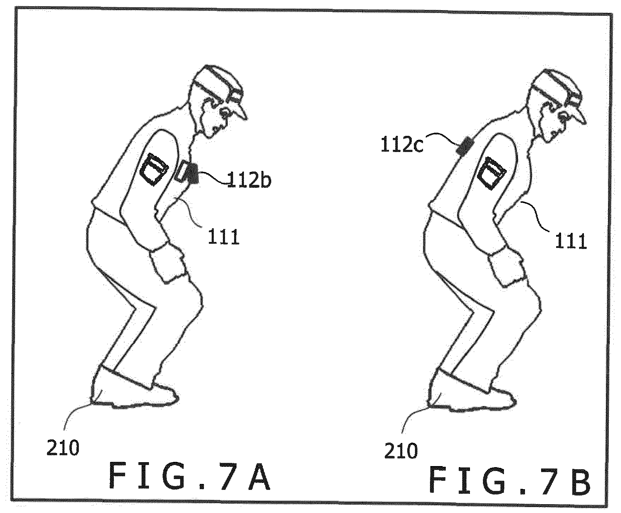

Figs. 7A and 7B are diagrams for describing a change in error according to the position of the posture sensor 112.

Fig. 7A illustrates an example in which the posture sensor 112b is arranged on the chest as in embodiment 1 (refer to Fig. 4D). In this case, when the user 210 tilts forward, the posture sensor 112b needs to tilt so as to accurately estimate the posture as the user 210 tilts forward. However, since the posture sensor 112b is attached to the clothing 111, the posture sensor 112b may become approximately vertical relative to the ground due to deformation of the chest of the clothing 111. In this case, there is a possibility that a large error may occur because no information can be acquired that indicates that the user 210 is tilting forward from the motion data 140 of the posture sensor 112b.

Fig. 7B illustrates an example in which the posture sensor is arranged on an upper back rather than on the chest. In this case, when the user 210 tilts forward, a posture sensor 112c which is the posture sensor 112 arranged on the upper back tilts forward as the user 210 tilts forward, which makes it unlikely that a large error will occur in the motion data 140 of the posture sensor 112c.

In the present embodiment, the posture estimation section 122 calculates posture data indicating the same posture of the user 210 for each posture sensor 112 on the basis of the motion data 140 measured by each of the plurality of posture sensors 112 (e.g., the posture sensors 112b and 112c illustrated in Figs. 7A and 7B) arranged on the plurality of different body parts of the user 210. The error estimation section 123 calculates, for each posture sensor 112, error data indicating an error that is occurring in that posture sensor 112. The information generation section 124 may generate additional information for each posture sensor 112 or may generate, as additional information, information obtained by comparing pieces of error data corresponding to the respective posture sensors 112 with each other.

It should be noted that although the plurality of posture sensors 112 are attached to different positions in the present embodiment, the posture sensors 112 may be attached in different manners in addition to or in place of the positions where the posture sensors 112 are attached. The manners in which the posture sensors 112 are attached include, for example, pasting to the clothing 111 with adhesive tape and embedding in the clothing 111 in advance.

According to the embodiment described above, it is possible to confirm how error data is changed according to the positions where the posture sensors 112 are attached and the manners in which the posture sensors 112 are attached. Accordingly, it is possible to find the position and manner in which the posture sensors 112 are attached that provide a small error, which makes it possible to realize the arrangement of the posture sensors 112 with a smaller error.

### [Embodiment 3]

In the present embodiment, a description will be given of an example of the posture recognition system 1 that can reduce an error by generating posture data and error data corresponding to each of the plurality of posture sensors 112 attached to different pieces of clothing. The posture sensors 112 are specifically attached to corresponding positions of respective pieces of the clothing 111 of the same type and different sizes. We assume here that the sizes are S, M, and L in the order from small to large. It should be noted, however, that the posture sensors 112 may be attached to different types of clothing.

The posture estimation section 122 calculates posture data indicating the same posture of the user 210 on the basis of the motion data 140 measured by each of the posture sensors 112 attached to the plurality of pieces of clothing 111, respectively, for each posture sensor 112 (i.e., for each piece of the clothing 111). Here, the same user 210 puts on the plurality of pieces of clothing 111 of different sizes in sequence and makes a given motion, and the posture estimation section 122 calculates feature quantities in sequence by calculating posture data corresponding to the respective pieces of the clothing 111 in sequence. It should be noted, however, that the posture estimation section 122 may calculate posture data in parallel for the plurality of pieces of clothing 111 put on by the different users 210. A single motion or a series of motions such as radio calisthenics may be made as a given motion.

The error estimation section 123 calculates, for each posture sensor 112, error data indicating an error that is occurring in that posture sensor 112. The information generation section 124 may generate additional information for each posture sensor 112 or may generate, as additional information, information obtained by comparing pieces of error data corresponding to the respective posture sensors 112 with each other.

According to the embodiment described above, it is possible to generate error data for each of the plurality of pieces of clothing. Accordingly, it becomes possible to select the piece of clothing 111 that suits the user 210 from among the plurality of pieces of clothing 111 such as selecting the piece of clothing 111 that provides the smallest error for the body shape of the user 120.

### [Embodiment 4]

In the present embodiment, a description will be given of an example of the posture recognition system 1 that can reduce an error by generating posture data and error data corresponding to each of the plurality of posture sensors 112 attached to different positions as in embodiment 2. It should be noted, however, that, in the present embodiment, an example will be described in which the posture sensors 112 are attached to the clothing 111 in such a manner as to be arranged on the same body part of the user 210.

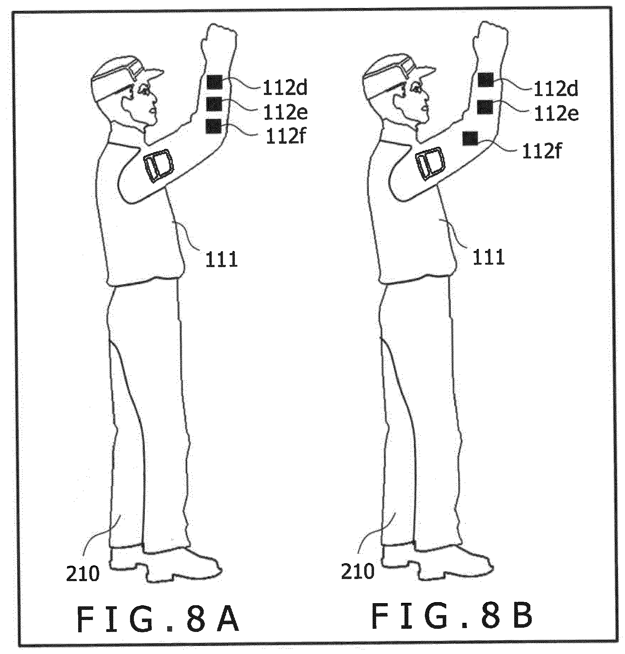

Figs. 8A and 8B are diagrams illustrating states in which the posture sensors 112 are attached to the clothing 111 in such a manner as to be arranged on the same body part of the user 210. The examples in Figs. 8A and 8B illustrate states in which the user 210 is wearing the clothing 111 to which three posture sensors 112d to 112f as the posture sensors 112 are attached at positions corresponding to the right forearm and in which the user has his or her arm raised.

In the example in Fig. 8A, even when the user 210 raises his or her arm, all the posture sensors 112d to 112f remain on the forearm. In this case, the values of error data based on the motion data 140 corresponding to the respective posture sensors 112d to 112f are equivalent.

In contrast, in the example in Fig. 8B, as the user 210 raises his or her arm, a sleeve of the clothing 111 slides down, which causes some of the posture sensors 112d to 112f (posture sensor 112f here) arranged on the forearm to move to the upper arm located more on the side of the shoulder than the forearm. In this case, if error data is calculated on the basis of the motion data 140 corresponding to each of the posture sensors 112d to 112f, the error data based on the motion data 140 of the posture sensor 112f that moves to the upper arm is larger than the error data based on the motion data 140 of the other posture sensors, namely, the posture sensors 112d and 112e.

In the present embodiment, the posture estimation section 122 calculates posture data indicating the same posture of the user 210 for each posture sensor 112 on the basis of the motion data 140 measured by each of the plurality of posture sensors 112 (e.g., the posture sensors 112d to 112f illustrated in Figs. 8A and 8B) arranged on the same body part of the user 210. The error estimation section 123 calculates, for each posture sensor 112, error data indicating an error that is occurring in that posture sensor 112. The information generation section 124 may generate additional information for each posture sensor 112 or may generate, as additional information, information obtained by comparing pieces of error data corresponding to the respective posture sensors 112 with each other.

It should be noted that although the plurality of posture sensors 112 are attached to the positions corresponding to the forearm in the present embodiment, the body part to which the posture sensors are attached is not limited to the forearm, and the posture sensors may be attached to a body part that can be considered a single rigid body (e.g., upper arm, forearm, shin).

As described above, it is possible, in the present embodiment, to realize more accurate posture recognition such as recognizing in what manner the clothing 111 is deformed.

The posture recognition system 1 in each of the embodiments described above is applicable, for example, to a work support system or an education system. The work support system can be used, for example, for training of maintenance tasks. The education system can be used, for example, for practicing dancing or yoga poses.

## Claims

1. A posture recognition system comprising:
a posture estimation section calculating, on a basis of sensor data measured by a posture sensor attached to clothing, posture data indicating a posture of a user wearing the clothing; and
an error estimation section calculating error data which is an estimate of an error that is occurring in the posture data, on a basis of the sensor data and the posture data.

2. The posture recognition system of claim 1, wherein
the posture estimation section generates, on a basis of sensor data measured by each of a plurality of the posture sensors, the posture data indicating the same posture of the user for each of the posture sensors, and
the error estimation section generates the error data for each of the posture sensors.

3. The posture recognition system of claim 2, wherein
the plurality of posture sensors are attached to the clothing in such a manner as to be arranged on a plurality of different body parts of the user.

4. The posture recognition system of claim 2, wherein
the plurality of posture sensors are attached to the clothing in such a manner as to be arranged on the same body part of the user.

5. The posture recognition system of claim 2, wherein
the plurality of posture sensors are attached to corresponding positions of the plurality of pieces of clothing.

6. The posture recognition system of claim 1, wherein
the posture data and the error data are chronological data.

7. The posture recognition system of claim 1 further comprising:
an information generation section generating additional information which is an evaluation of the error data.

8. A posture recognition method by a posture recognition system, the posture recognition method comprising:
calculating, on a basis of sensor data measured by a posture sensor attached to clothing, posture data indicating a posture of a user wearing the clothing; and
calculating error data which is an estimate of an error that is occurring in the posture data, on a basis of the sensor data and the posture data.

9. A program causing a computer to perform:
calculating, on a basis of sensor data measured by a posture sensor attached to clothing, posture data indicating a posture of a user wearing the clothing; and
calculating error data which is an estimate of an error that is occurring in the posture data, on a basis of the sensor data and the posture data.
